# EUROPEAN PATENT SPECIFICATION

(11) **EP 3 669 810 B1**
(45) Date of publication and mention of the grant of the patent: **13.03.2024**
(21) Application number: 19216994.4
(22) Date of filing: 17.12.2019
(51) Int. Cl.: A61B 34/00, A61M 25/09, H01F 1/055, H01F 1/057, A61B 34/30, A61M 25/01

(54) **MICRO-ROBOT FOR STEERING GUIDEWIRE**
MIKROROBOTER FÜR LENKFÜHRUNGSDRAHT
MICRO-ROBOT POUR DIRIGER UN FIL-GUIDE

(30) Priority: 21.12.2018 KR 20180167889
(43) Date of publication of application: 24.06.2020
(73) Proprietor: Daegu Gyeongbuk Institute of Science and Technology, Dalseong-gun, Daegu 42988 (KR)
(72) Inventor: CHOI, Hong Soo, Daegu 42951 (KR); KIM, Jin Young, Suwon-si Gyeonggi-do 16682 (KR); LEE, Sun Key, Daegu 42995 (KR); KIM, Kang Ho, Busan 48420 (KR)
(74) Representative: Grünecker Patent- und Rechtsanwälte PartG mbB

(56) References cited:
- EP-A1- 3 181 176
- EP-A1- 3 348 301
- CN-B- 101 783 219
- JP-A- 2005 057 023
- US-A1- 2004 133 130
- US-A1- 2006 144 407

## Description

### BACKGROUND

### 1. Field

The disclosure relates to a micro-robot, and more particularly, to a micro-robot for steering a guidewire.

### 2. Description of Related Art

When lesions are present inside a body, a guidewire is inserted in a blood vessel until reaching a region where the lesions are located along the blood vessel to treat the lesions. However, making a guidewire reach a region where lesions are located without damaging the blood vessel is a difficult task requiring high professional skills.

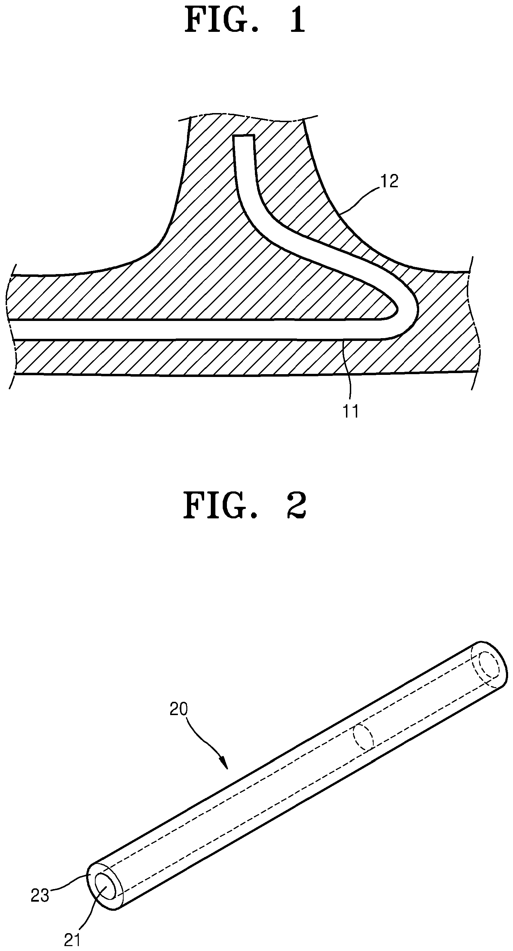

In some cases, the guidewire should be steered at a great angle to enter the next blood vessel. However, when the steering angle range of the guidewire 11 is insufficient, buckling in which a portion of the guidewire is excessively bent may occur, as shown in FIG. 1, which may damage a blood vessel 12.

Also, in some cases, the guidewire should be replaced with another one depending on a degree of hardness, etc. of a blood clot. In these cases, a task of replacing the guidewire with another one and then again inserting it into a blood vessel is required, which increases damage probability of the blood vessel.

US2004/0133130 A1 discloses a magnetic guidewire according to the prior art.

### SUMMARY

By mounting a micro-robot on a guidewire, the guidewire may be steered in a wide range. By mounting a micro-robot on a guidewire, the guidewire may be used without being replaced with another one for different degrees of hardness of blood clots.

It should be noted that objects of the present disclosure are not limited to the above-described objects, and other objects of the present disclosure will be apparent to those skilled in the art from the following descriptions.

Additional aspects will be set forth in part in the description which follows and, in part, will be apparent from the description, or may be learned by practice of the presented embodiments of the disclosure.

The invention is defined in appended independent claim 1, further embodiments are described in the dependent claims.

A micro-robot capable of being mounted on a guidewire, according to an embodiment of the disclosure, includes: a first magnetic body; and a second magnetic body positioned in one direction from the first magnetic body, wherein the first magnetic body is more flexible than the second magnetic body, and the first magnetic body is capable of bending at an angle that changes according to a change in a direction of an external magnetic field.

In the micro-robot, the second magnetic body may maintain the same shape regardless of the change in the direction of the external magnetic field.

In the micro-robot, the first magnetic body may include at least one among praseodymium-iron-cobalt alloy powder and neodymium-iron-boron alloy powder.

In the micro-robot, the first magnetic body may include polydimethylsiloxane.

In the micro-robot, a width of the micro-robot may be about 0.5 mm or less.

In the micro-robot, a width of the micro-robot may be uniform.

In an embodiment not according to the invention, when the micro-robot is mounted on the guidewire, a portion of the guidewire may be positioned inside the micro-robot, and the remaining portion of the guidewire may be positioned in an opposite direction to the one direction from the first magnetic body.

In the micro-robot, one of the first magnetic body and the second magnetic body may be mounted on the guidewire to surround an end of the guidewire.

The micro-robot may further include a biocompatible tube surrounding at least one portion of the first magnetic body and the second magnetic body, and including a biocompatibility material.

In the micro-robot, the second magnetic body may be adjacent to the first magnetic body.

In the micro-robot, a length of the first magnetic body may be longer than a length of the second magnetic body.

Stiffness of the micro-robot may increase as an intensity of the external magnetic field increases.

### BRIEF DESCRIPTION OF THE DRAWINGS

The above and other aspects, features, and advantages of certain embodiments of the disclosure will be more apparent from the following description taken in conjunction with the accompanying drawings, in which:
FIG. 1 shows occurrence of buckling in a guidewire inserted in a blood vessel;
FIGS. 2 and 3 show an example of a micro-robot according to an embodiment;
FIG. 4 shows an example of a micro-robot mounted on a guidewire according to an embodiment;
FIG. 5 shows measured steering angles of a micro-robot, according to an experimental example;
FIGS. 6A and 6B are views for comparing steering angles of a guidewire with a micro-robot with steering angles of another guidewire, according to an experimental example;
FIGS. 7A and 7B are views for comparing a steering angle of a guidewire with a micro-robot with a steering angle of still another guidewire, according to an experimental example; and
FIGS. 8A and 8B show measured stiffness of a micro-robot, according to an experimental example.

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. As used herein, the term "and/or" includes any and all combinations of one or more of the associated listed items. Expressions such as "at least one of," when preceding a list of elements, modify the entire list of elements and do not modify the individual elements of the list.

In the present specification, the terms "configured" or "including" should not be construed as necessarily including all of various components or operations described in the specification. It should be construed that some of the components or operations are omitted or that additional components or operations may be further included.

Although general terms being widely used at the disclosure were selected as terminology used in the specification while considering the functions of the disclosure, they may vary according to intentions of one of ordinary skill in the art, judicial precedents, the advent of new technologies, and the like. Terms arbitrarily selected by the applicant of the disclosure may also be used in a specific case. In this case, their meanings need to be given in the detailed description of the disclosure. Hence, the terms must be defined based on the meanings of the terms and the contents of the entire specification, not by simply stating the terms themselves.

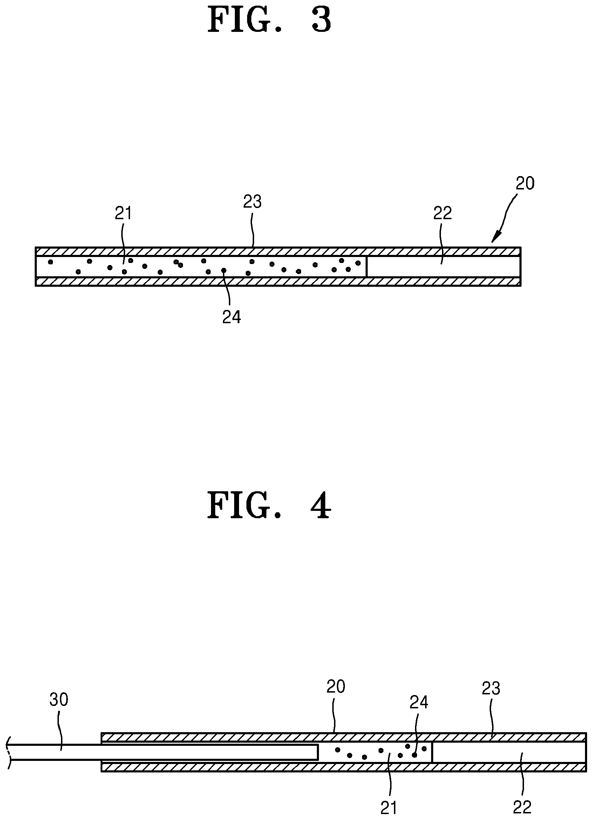

FIGS. 2 and 3 show an example of a micro-robot according to an embodiment.

A micro-robot 20 may be in the shape of a cylinder with a uniform width. The width of the micro-robot 20 may be in a range of about 0.2 mm to about 1 mm. The width of the micro-robot 20 may be in a range of about 0. 2 mm to about 0. 5 mm, although not limited thereto. However, the micro-robot 20 may be in another shape, such as a cone, a polyprism, etc. That is, the shape of the micro-robot 20 is not limited to the shape shown in FIGS. 2 and 3.

The micro-robot 20 may include a first magnetic body 21, a second magnetic body 22 and a biocompatible tube 23.

The first magnetic body 21 may include a plurality of magnetic particles 24. For example, the first magnetic body 21 may include at least one among praseodymium-iron-cobalt (PrFeCo) alloy powder, neodymium-iron-boron (NdFeB) alloy powder, iron powder, iron oxide powder and nickel powder. The first magnetic body 21 may further include other magnetic particles. Also, the first magnetic body 21 may include at least one among polydimethylsiloxane (PDMS), gelatin and agarose gel. The first magnetic body 21 may further include other polymers having biocompatibility.

For example, the first magnetic body 21 may be manufactured by mixing praseodymium-iron-cobalt alloy powder and neodymium-iron-boron alloy powder with polydimethylsiloxane and then hardening the mixture at high temperature.

A length of the first magnetic body 21 may be in a range of about 5 mm to about 100 mm. A length of the first magnetic body 21 may be in a range of about 5 mm to 50 mm, although not limited thereto.

A width of the first magnetic body 21 may be in a range of about 0.1 mm to about 1 mm. A width of the first magnetic body 21 may be in a range of about 0.1 mm to about 0.5 mm, although not limited thereto.

The second magnetic body 22 may be positioned in one direction from the first magnetic body 21. For example, the second magnetic body 22 may be adjacent to the first magnetic body 21. As another example, the second magnetic body 22 may be spaced from the first magnetic body 21.

The second magnetic body 22 may include a neodymium-iron-boron alloy.

A length of the second magnetic body 22 may be in a range of about 0.1 mm to about 50 mm. A length of the second magnetic body 22 may be in a range of about 0.1 mm to about 30 mm, although not limited thereto.

A width of the second magnetic body 22 may be the same as the width of the first magnetic body 21. Alternatively, the second magnetic body 22 may have a smaller diameter at a longer distance from the first magnetic body 21.

The first magnetic body 21 may be more flexible than the second magnetic body 22. Accordingly, when an external magnetic field is applied, the first magnetic body 21 may be bent, but the second magnetic body 22 may maintain the same shape without being bent.

The biocompatible tube 23 may surround at least one portion of the first magnetic body 21 and the second magnetic body 22. In FIGS. 2 and 3, the biocompatible tube 23 surrounds the entire of the first magnetic body 21 and the second magnetic body 22. On the contrary, the biocompatible tube 23 may surround a portion of the first magnetic body 21 and the entire of the second magnetic body 22, or the biocompatible tube 23 may surround the entire of the first magnetic body 21 and a portion of the second magnetic body 22.

The biocompatible tube 23 may include, as the name suggests, a biocompatibility material. For example, the biocompatible tube 23 may include silicon, gore-tex, natural rubber, polymethylmethacrylate (PMMA), polyhydroxyethylmethacrylate (PHEMA), polyethylene terephthalate (PET) and a combination thereof.

A difference between an internal diameter and an external diameter of the biocompatible tube 23, that is, a thickness of the biocompatible tube 23 may be in a range of about 0.01 mm to about 1 mm, although not limited thereto. A thick biocompatible tube may function as a resistant element of reducing a degree of bending (flexibility) of a micro-robot. Also, a thick biocompatible tube may function as an element of increasing stiffness of a micro-robot. Accordingly, the width of the biocompatible tube 23 may be selected as an appropriate thickness such that the micro-robot 20 has appropriate stiffness and flexibility.

Also, the stiffness of the biocompatible tube 23 may depend on a biocompatibility material included therein. Accordingly, the biocompatible tube 23 may be manufactured with an appropriate biocompatibility material such that the micro-robot 20 has appropriate stiffness and flexibility.

When the biocompatible tube 23 surrounds the first magnetic body 21 and the second magnetic body 22, the micro-robot 20 may be safely inserted in an inside of a body.

FIG. 4 shows an example which is not according to the invention of a micro-robot mounted on a guidewire.

The micro-robot 20 may be mounted on a guidewire 30. The micro-robot 20 may be mounted on the guidewire 30 such that the first magnetic body 21 forms a proximal portion with respect to the guidewire 30 and the second magnetic body 22 forms a distal portion with respect to the guidewire 30.

A portion of the guidewire 30 may be inserted in the micro-robot 20. A length of the portion of the guidewire 30 inserted in the micro-robot 20 may be in a range of about 5 mm to about 70 mm. A length of the portion of the guidewire 30 inserted in the micro-robot 20 may be in a range of about 5 mm to about 50 mm, although not limited thereto.

The micro-robot 20 may be mounted on the guidewire 30 such that the first magnetic body 21 surrounds an end of the guidewire 30. For example, by inserting an end of the guidewire 30, together with materials (for example, praseodymium-iron-cobalt alloy powder, neodymium-iron-boron alloy powder, or polydimethylsiloxane) for manufacturing the first magnetic body 21, into a space surrounded by the biocompatible tube 23 and then hardening the materials, the first magnetic body 21 may be coupled to the guidewire 30.

According to the invention, the micro-robot 20 may be mounted on the guidewire 30 such that the second magnetic body 22 surrounds an end of the guidewire 30. For example, by inserting an end of the guidewire 30 into a groove formed in the second magnetic body 22 and then fixing the end of the guidewire 30 with an adhesive, the second magnetic body 22 may be coupled to the guidewire 30.

A diameter of the micro-robot 20 may be similar to a diameter of the guidewire 30. For example, a difference between the diameter of the micro-robot 20 and the diameter of the guidewire 30 may be in a range of about 0 mm to about 0.1 mm.

Because the diameter of the micro-robot 20 is similar to the diameter of the guidewire 30, the guidewire 30 may be inserted into a blood vessel without being interfered by a size of the micro-robot 20.

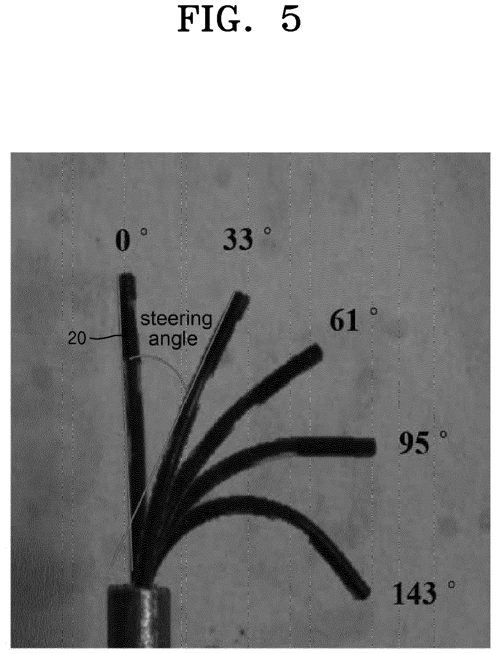

FIG. 5 shows measured steering angles of a micro-robot, according to an experimental example.

According to an experimental example, an external magnetic field may be applied in various directions to the micro-robot 20. FIG. 5 shows steered states of the micro-robot 20 when an external magnetic field is applied in different directions. When an external magnetic field is applied, steering angles of 33°, 61°, 95° and 143° may be acquired.

Because the micro-robot 20 according to an embodiment of the disclosure includes the first magnetic body 21 having flexibility, a wide range of steering angles may be acquired when the first magnetic body 21 is bent by an external magnetic field. Accordingly, by mounting the micro-robot 20 according to an embodiment of the disclosure on the guidewire 30, the guidewire 30 may also be steered to a wide range of angles.

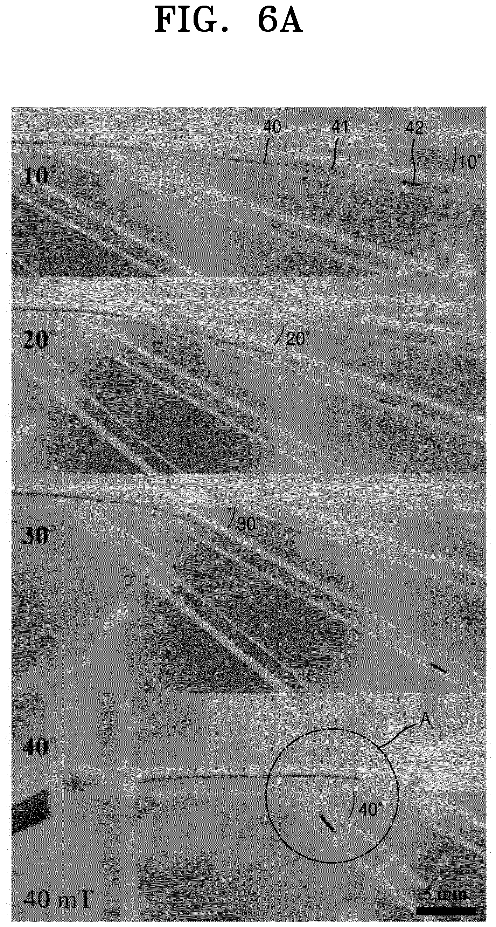

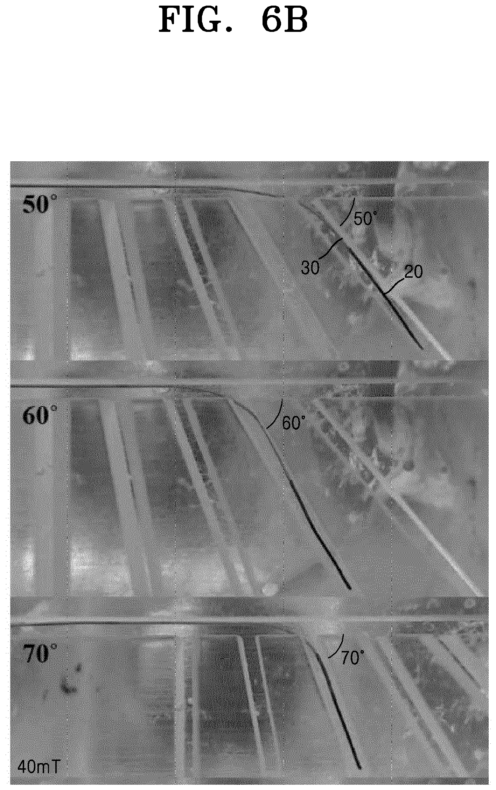

FIGS. 6A and 6B are views for comparing steering angles of a guidewire with a micro-robot to steering angles of another guidewire, according to an experimental example.

FIG. 6A shows a range of steering angles of another guidewire 40. A flexible material 41 having no magnetism and a magnetic body 42 having no flexibility were mounted on an end of the other guidewire 40. According to an experimental example, the other guidewire 40 was steered to 10°, 20° and 30°. However, when the other guidewire 40 was steered to 40°, buckling occurred.

Meanwhile, referring to FIG. 6B, the guidewire 30 on which the micro-robot 20 is mounted was steered to 50°, 60° and 70°. Accordingly, it is seen that, when the first magnetic body 21 having flexibility is used, the guidewire 30 is steered to a wider angle than when the flexible material 41 having no magnetism is used.

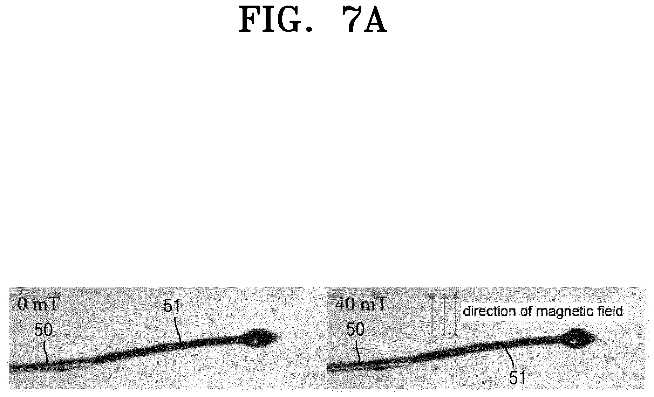

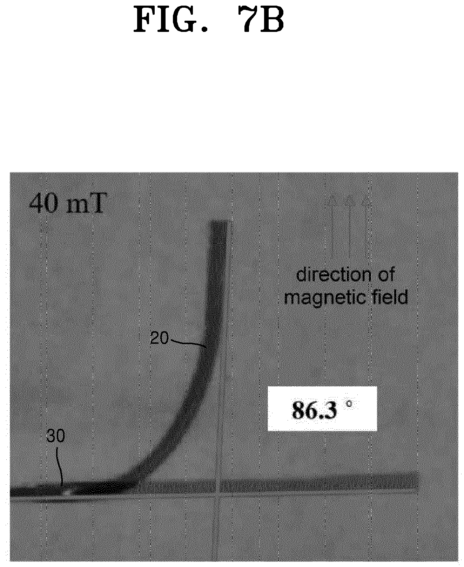

FIGS. 7A and 7B are views for comparing a steering angle of a guidewire with a micro-robot to a steering angle of still another guidewire, according to an experimental example.

FIG. 7A shows still another guidewire before and after an external magnetic field is applied. A magnetorheological fluid 51 was mounted on an end of the still another guidewire 50. It is seen that the still another guidewire 50 little moves even when an external magnetic field of 40mT is applied thereto.

Meanwhile, referring to FIG. 7B, the guidewire 30 on which the micro-robot 20 is mounted was steered to 86.3° when an external magnetic field of 40mT was applied thereto. Accordingly, it is seen that, when the micro-robot 20 according to an embodiment of the disclosure is used, the guidewire 30 is more easily steered than when the magnetorheological fluid 51 is used.

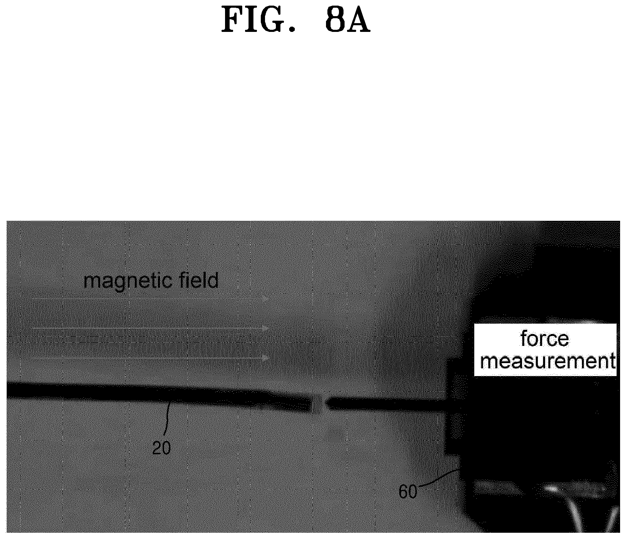

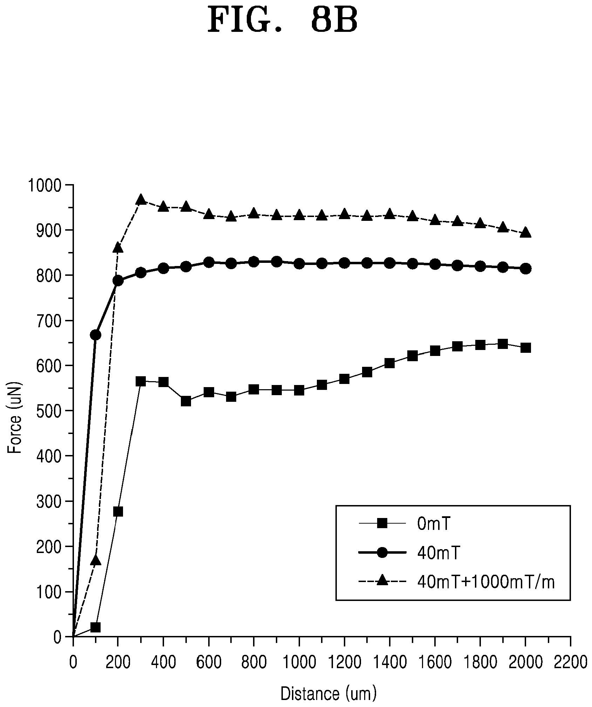

FIGS. 8A and 8B show measured stiffness of a micro-robot, according to an experimental example.

According to an experimental example, external magnetic fields of various intensities were applied to the micro-robot 20. A graph of FIG. 8B shows forces applied to a force sensor 60 by an end of the micro-robot 20 when external magnetic fields of different intensities are applied. Referring to the graph, it is seen that, the stronger external magnetic field is applied, the greater force sensed by the force sensor 60, which indicates an increase in stiffness of the micro-robot 20. Accordingly, because the stiffness of the micro-robot 20 is adjustable by adjusting an intensity of an external magnetic field, the guidewire 30 may be used without being replaced with another one according to degrees of hardness of blood clots.

The guidewire 30 may be steered to a wide range of angles. Accordingly, buckling may be prevented from occurring in a blood vessel, and a blood vessel may be prevented from being damaged by the guidewire 30. Also, because the stiffness of the micro-robot 20 is adjustable, the guidewire 30 may be used without being replaced with another one according to degrees of hardness of blood clots.

The effect of the disclosure is not limited to the content exemplified above, and more various effects may be included in this specification.

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by those of ordinary skill in the art that various changes in form and details may be made therein without departing from the scope of the disclosure as defined by the following claims.

## Claims

1. A combination of a micro-robot (20) and a guidewire (30), the micro-robot (20) capable of being mounted on the guidewire (30), the micro-robot (20) comprising:
a first magnetic body (21); and
a second magnetic body (22) positioned in one direction from the first magnetic body (21), wherein
the first magnetic body (21) is more flexible than the second magnetic body (22), and
the first magnetic body (21) is capable of bending at an angle that changes according to a change in a direction of an external magnetic field;
**the** combination being **characterized in that**:
when the micro-robot (20) is mounted on the guidewire (30), a portion of the guidewire (30) is
positioned inside the micro-robot (20) and the remaining portion of the guidewire (30) is positioned in the one direction from the first magnetic body (21).

2. The combination of claim 1, wherein the first magnetic body (21) comprises at least one among praseodymium-iron-cobalt alloy powder, neodymium-iron-boron alloy powder, iron powder, iron oxide powder, and nickel powder.

3. The combination of claim 1, wherein the first magnetic body (21) comprises at least one among polydimethylsiloxane, gelatin, and agarose gel.

4. The combination of claim 1, wherein a width of the micro-robot (20) is about 0.5 mm or less.

5. The combination of claim 1, wherein a width of the micro-robot (20) is uniform.

6. The combination of claim 1, wherein one of the first magnetic body (21) and the second magnetic body (22) is mounted on the guidewire (30) to surround an end of the guidewire (30).

7. The combination of claim 1, further comprising a biocompatible tube (23) surrounding at least one portion of the first magnetic body (21) and the second magnetic body (22) and comprising a biocompatibility material.

8. The combination of claim 1, wherein the second magnetic body (22) is adjacent to the first magnetic body (21).

9. The combination of claim 1, wherein stiffness of the micro-robot (20) increases as an intensity of the external magnetic increases.

## Patentansprüche

1. Eine Kombination eines Mikroroboters (20) und eines Führungsdrahtes (30), wobei der Mikroroboter (20) auf dem Führungsdraht (30) montiert werden kann, der Mikroroboter (20) umfassend:
einen ersten Magnetkörper (21); und
einen zweiten Magnetkörper (22), der in einer Richtung von dem ersten Magnetkörper (21) positioniert ist, wobei
der erste Magnetkörper (21) flexibler ist als der zweite Magnetkörper (22), und
der erste Magnetkörper (21) in der Lage ist, sich in einem Winkel zu biegen, der sich entsprechend einer Richtungsänderung eines externen Magnetfeldes ändert;
**die Kombination dadurch gekennzeichnet, dass**
wenn der Mikroroboter (20) auf dem Führungsdraht (30) montiert ist, ein Teil des Führungsdrahtes (30) innerhalb des Mikroroboters (20) positioniert ist und der verbleibende Teil des Führungsdrahtes (30) in der einen Richtung von dem ersten Magnetkörper (21) positioniert ist.

2. Die Kombination nach Anspruch 1, wobei der erste Magnetkörper (21) mindestens eines unter Praseodym-Eisen-Kobalt-Legierungspulver, Neodym-Eisen-Bor-Legierungspulver, Eisenpulver, Eisenoxidpulver und Nickelpulver umfasst.

3. Die Kombination nach Anspruch 1, wobei der erste Magnetkörper (21) mindestens eines unter Polydimethylsiloxan, Gelatine und Agarosegel umfasst.

4. Die Kombination nach Anspruch 1, wobei die Breite des Mikroroboters (20) etwa 0,5 mm oder weniger beträgt.

5. Die Kombination nach Anspruch 1, wobei die Breite des Mikroroboters (20) gleichmäßig ist.

6. Die Kombination nach Anspruch 1, wobei entweder der erste Magnetkörper (21) oder der zweite Magnetkörper (22) auf dem Führungsdraht (30) angebracht ist, um ein Ende des Führungsdrahtes (30) zu umgeben.

7. Die Kombination nach Anspruch 1, femer ein biokompatibles Rohr (23) umfassend, das mindestens einen Teil des ersten Magnetkörpers (21) und des zweiten Magnetkörpers (22) umgibt und ein biokompatibles Material umfasst.

8. Die Kombination nach Anspruch 1, wobei der zweite Magnetkörper (22) an den ersten Magnetkörper (21) angrenzt.

9. Die Kombination nach Anspruch 1, wobei die Steifigkeit des Mikroroboters (20) mit zunehmender Intensität des externen Magnetfelds zunimmt.

## Revendications

1. Combinaison d'un micro-robot (20) et d'un fil de guidage (30), le micro-robot (20) capable d'être monté sur le fil de guidage (30), le micro-robot (20) comprenant :
un premier corps magnétique (21) ; et
un second corps magnétique (22) positionné dans un sens depuis le premier corps magnétique (21),
le premier corps magnétique (21) étant plus souple que le second corps magnétique (22), et
le premier corps magnétique (21) étant capable de flexion sous un angle qui change en fonction d'un changement dans une direction d'un champ magnétique externe ;
la combinaison étant **caractérisée en ce que** :
lorsque le micro-robot (20) est monté sur le fil de guidage (30), une portion du fil de guidage (30) est positionnée à l'intérieur du micro-robot (20) et la portion restante du fil de guidage (30) est positionnée dans un sens du premier corps magnétique (21).

2. Combinaison selon la revendication 1, le premier corps magnétique (21) comprenant au moins l'un parmi une poudre d'alliage de praséodymium-fer-cobalt, une poudre d'alliage de néodymium-fer-bore, une poudre de fer, une poudre d'oxyde de fer, et une poudre de nickel.

3. Combinaison selon la revendication 1, le premier corps magnétique (21) comprenant au moins l'un parmi le polydiméthylsiloxane, la gélatine, et un gel d'agarose.

4. Combinaison selon la revendication 1, une largeur du micro-robot (20) étant d'environ 0,5 mm ou moins.

5. Combinaison selon la revendication 1, une largeur du micro-robot (20) étant uniforme.

6. Combinaison selon la revendication 1, l'un du premier corps magnétique (21) et du second corps magnétique (22) étant monté sur le fil de guidage (30) pour entourer une extrémité du fil de guidage (30).

7. Combinaison selon la revendication 1, comprenant en outre un tube biocompatible (23) entourant au moins une portion du premier corps magnétique (21) et du second corps magnétique (22) et comprenant un matériau à biocompatibilité.

8. Combinaison selon la revendication 1, le second corps magnétique (22) étant adjacent au premier corps magnétique (21).

9. Combinaison selon la revendication 1, une rigidité du micro-robot (20) croissant lorsqu'une intensité du magnétique externe augmente.
